# EUROPEAN PATENT APPLICATION

(11) **EP 2 128 614 A1**
(43) Date of publication of application: **02.12.2009**
(21) Application number: 09160095.7
(22) Date of filing: 13.05.2009
(51) Int. Cl.: G01N 33/53, C12Q 1/68, B01L 3/00

(54) **Microfluidic device containing lyophilized reagent therein and analyzing method using the same**

(30) Priority: 14.05.2008 KR 20080044723
(71) Applicant: Samsung Electronics Co., Ltd., Suwon-si Gyeonggi-do (KR)
(72) Inventor: Cho, Yoon-kyoung, Gyeonggi-do (KR); Lee, Jeong-gun, Gyeonggi-do (KR)
(74) Representative: Grünecker, Kinkeldey, Stockmair & Schwanhäusser Anwaltssozietät

(57) **Abstract**

Provided is a microfluidic device suitable for analyzing a liquid sample. The device includes a substrate comprising a plurality of chambers; a solid reagent contained in at least one of the plurality of chambers, wherein at least a portion of the shape of the solid reagent is identical to at least a portion of the configuration of the inner surface of the at least one chamber.

## Description

### BACKGROUND

### 1. Field of the Invention

One or more embodiments related to a microfluidic device containing a reagent and a method of analyzing a sample using the microfluidic device, and more particularly, to a microfluidic device containing a predetermined amount of a lyophilized reagent and a method of analyzing a sample using the microfluidic device.

### 2. Description of the Related Art

Various methods of analyzing a sample have been developed to, for example, monitor environments, examine food, or diagnose the medical condition of a patient. However, these methods require many steps pf manual operations and various devices. To perform an examination according to a predetermined protocol, those skilled in the manual operations repeatedly perform various processes including loading a reagent, mixing, isolating and transporting, reacting, and centrifuging. Such manually operated, repeated processes, however, often cause erroneous results due to "human errors."

To perform examinations quickly, skilled clinical pathologists are needed. However, it is hard for even a skilled clinical pathologist to perform various examinations simultaneously. Also, rapid examination results are essential for immediate timely treatments of emergency patients. Accordingly, there is a need to develop various types of equipment enabling simultaneous, rapid and accurate pathological examinations for given circumstances.

Conventional pathological examinations are performed with large and expensive pieces of automated equipment and a relatively large amount of a sample, such as blood. Moreover, it usually takes from 2 days (at the minimum) to about 2 weeks to obtain results of pathological examinations after collecting a blood sample from a patient.

In order to solve the above described problems, small and automated pieces of equipment for analyzing a sample taken from one or, if needed, a small number of patients over a short time period have been developed. An example of such a system involves the use of a microfluidic device. In a microfluidic device, a blood sample is loaded into the disc-shaped microfluidic device and the disc-shaped microfluidic device is rotated, and then serum can be isolated from the blood sample due to the centrifugal force. The isolated serum is mixed with a predetermined amount of a diluent and the resulting mixture then flows to a reaction chamber (usually, plural reaction chambers are provided) in the disc-shaped microfluidic device. The reaction chambers are filled with a reagent prior to allowing the mixture to flow therein. The regent used may differ according to of the goal of the tests. When serum is brought into contact with different reagents, the mixture of the serum and the reagent may change its color. The change in color is used to perform a quantitative and qualitative analysis of a blood sample.

It is difficult to store a reagent of a liquid state in chambers of the microfluidic device. US Patent 5,776,563 discloses a system in which various kinds of reagents are lyophilized and formed into beads each of a predetermined amount. Then just prior to performing a blood analysis, the lyophilized reagents are loaded in a required amount to reaction chambers of the microfluidic device.

### SUMMARY

One or more embodiments provide a microfluidic device containing a lyophilized reagent in a certain amount and a method of analyzing a biological sample using the microfluidic device.

In an exemplary embodiment, there is provided a microfluidic device including: a substrate comprising a plurality of chambers; a solid reagent contained in at least one of the plurality of chambers, wherein at least a portion of the shape of the solid reagent is identical to at least a portion of the configuration of the inner surface of the at least one chamber.

In an embodiment, the solid reagent may be lyophilized reagent.

In an embodiment, the microfluidic device may further comprises a plurality of channels connecting the plurality of chambers; and a valve, included in at least one of the plurality of channels, which controls flow of a fluid through the plurality of channels.

The valve may be formed of a valve forming material that changes state when exposed to electromagnetic radiation such that the valve opens. The valve forming material is selected from a phase transition material and a thermoplastic resin, wherein the phase of the phase transition material or the thermoplastic resin changes when exposed to energy of the electromagnetic radiation. The phase transition material is selected from wax and gel. The valve forming material includes heat dissipating particles which are dispersed in the phase transition material, and absorb energy of the electromagnetic radiation and dissipate the energy. The heat dissipating particles are selected from particles of metal oxides, polymer particles, quantum dots, and magnetic beads.

In an embodiment, the first reagent may be selected from buffer and distilled water.

In an embodiment, the solid reagent includes at least one reagent selected from the group consisting of reagents for detecting serum, aspartate aminotransferase (AST), albumin (ALB), alkaline phosphatase (ALP), alanine aminotransferase (ALT), amylase (AMY), urea nitrogen (BUN), calcium (Ca++), total cholesterol (CHOL), creatin kinase (CK), chloide (Cl-), creatinine (CREA), direct bilirubin (D-BIL), gamma glutamyl transferase (GGT), glucose (GLU), high-density lipoprotein cholesterol (HDL), potassium (K+), lactate dehydrogenase (LDH), low-density lipoprotein cholesterol (LDL), magnesium (Mg), phosphorus (PHOS), sodium (Na+), total carbon dioxide (TCO₂) total bilirubin (T-BIL), triglycerides (TRIG), uric acid (UA), albumin (ALB), or total protein (TP).

In an embodiment, the solid reagent may include a filler. The filler includes at least one material selected from the group consisting of bovine serum albumin (BSA), polyethylene glycol (PEG), dextran, mannitol, polyalcohol, myo-inositol, an citric acid, ethylene diamine tetra acetic acid disodium salt (EDTA2Na), and polyoxyethylene glycol dodecyl ether (BRIJ-35).

In an embodiment, the solid reagent may include a surfactant. The surfactant includes at least one material selected from the group consisting of polyoxyethylene, lauryl ether, octoxynol, polyethylene alkyl alcohol, nonylphenol polyethylene glycol ether; ethylene oxid, ethoxylated tridecyl alcohol, polyoxyethylene nonylphenyl ether phosphate sodium salt, and sodium dodecyl sulfate.

In an embodiment, the solid reagent may be prepared by condensing a second reagent to have a concentration higher than a concentration that is used for an examination and lyophilizing the condensed second reagent.

In an embodiment, the plurality of chambers may comprise a first chamber to contain a liquid sample to be analyzed, a second chamber to contain a liquid reagent, and a third chamber which contains the solid reagent.

In an embodiment, the third chamber may be a detection chamber that is used to detect a specific material included in the sample. The detection chamber is a transparent chamber.

In an embodiment, the third chamber may include a plurality of sub-chambers, wherein a plurality of solid reagent components are respectively contained in a lyophilized state in the plurality of sub-chambers, wherein when the plurality of solid reagents components are mixed and lyophilized, activity of the plurality of solid reagent components degrades.

In an embodiment, the microfluidic device may further include a transparent detection chamber connected to the third chamber, wherein the third chamber is non-transparent so that light does not pass through the third chamber.

In an embodiment, the microfluidic device may further include a sample discharge chamber that is connected to the first chamber and accommodates excess sample.

In an embodiment, the microfluidic device may further include a liquid reagent discharge chamber that is connected to the second chamber and accommodates excess liquid reagent.

According to another aspect of the present invention, there is provided a method of analyzing a sample using a microfluidic device comprising plural chambers connected by a plurality of channels each comprising a valve, the method comprising: providing a microfluidic device of which chamber (I) contains a solid lyophilized reagent (I); loading a liquid reagent (II) into a chamber (II); loading the sample into a chamber (III); opening the valve and mixing the sample with the reagent (II) to form a sample mixture; mixing the sample mixture with the lyophilized reagent (I) to form a reagent mixture; and detecting a reaction of the reagent mixture in the chamber (I).

In an embodiment, at least a portion of the shape of the lyophilized second reagent is identical to at least a portion of the shape of the chamber (I).

In an embodiment, the opening of the valve includes supplying electromagnetic energy to a valve forming material in the channel so that the valve forming material melts.

### BRIEF DESCRIPTION OF THE DRAWINGS

The above and other aspects of the present invention will become more apparent by describing in detail exemplary embodiments thereof with reference to the attached drawings in which:

FIG. 1 is a plan view of a microfluidic device according to an embodiment of the present invention;

FIG. 2 is a cross-sectional view of the microfluidic device of FIG. 1 as a two-layered microfluidic device according to an embodiment of the present invention;

FIG. 3 is a cross-sectional view of the microfluidic device of FIG. 1 as a three-layered microfluidic device according to another embodiment of the present invention;

FIG. 4 shows a schematic view of an analyzer including the microfluidic device of FIG 1;

FIG. 5 illustrates a cross-sectional view of a channel that is opened when a valve melts;

FIG. 6 is a plan view of a microfluidic device according to another embodiment of the present invention;

FIG. 7 is a plan view of a microfluidic device according to another embodiment of the present invention;

FIG. 8 is a plan view of a microfluidic device according to another embodiment of the present invention;

FIG. 9 is a plan view of a microfluidic device according to another embodiment of the present invention;

FIG. 10 is a plan view of a microfluidic device according to another embodiment of the present invention; and

FIG. 11 is a plan view of a microfluidic device according to another embodiment of the present invention.

### DETAILED DESCRIPTION

The present invention will now be described more fully with reference to the accompanying drawings, in which exemplary embodiments of the invention are shown.

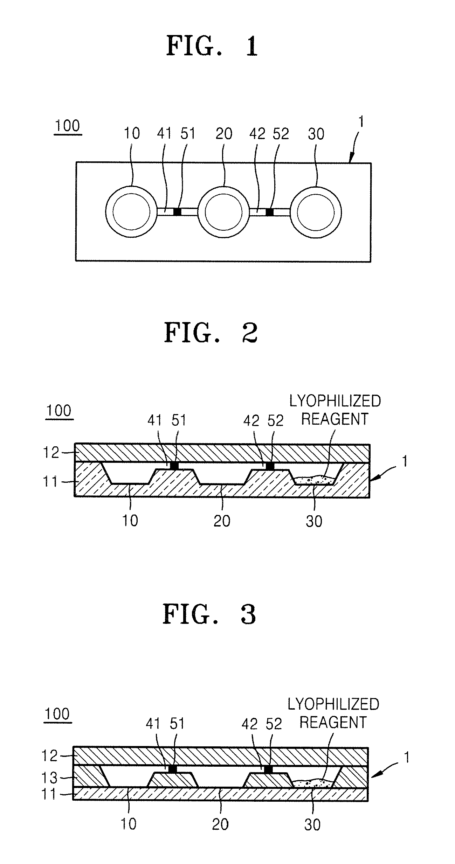

FIG. 1 is a plan view of a microfluidic device 100 according to an embodiment of the present invention, and FIGS. 2 and 3 are cross-sectional views of the microfluidic device 100 of FIG. 1, according to two different embodiments of the present invention.

Referring to FIGS. 1 and 2, the microfluidic device 100 has a substrate 1 having a microfluidic structure including a portion which is configured for storing a fluid and a channel through which the fluid flows. The substrate 1 may be formed of a plastic material that can be easily molded and is biologically inactive. The plastic material may be acryl or polydimethylsiloxane (PDMS). However, a material for forming the substrate 1 is not limited thereto described above and can be any material that is chemically and biologically stable and optically transparent, as well as has a mechanical processability. The substrate 1 may have, as illustrated in FIG. 2, a two-layer structure including a first plate 11 and a second plate 12. The substrate 1 can also have, as illustrated in FIG 3, a three-layered structure including a first plate 11, a second plate 12, and an intermediate plate 13 disposed between the first plate 11 and the second plate 12. Throughout the disclosure, the first and the second plates are sometimes identified as "bottom plate" and "top plate," respectively, for purpose of explaining them as depicted in the drawings. The intermediate plate 13 is formed to define a portion for storing a fluid and a channel through which the fluid flows. The bottom plate 11, the top plate 12, and the intermediate plate 13 can be bonded together using various methods, such as using a double-sided tape or an adhesive, or fusing using supersonic waves. The substrate 1 can also have other structures as long as it provides channels and compartments (or chambers) configured for biochemical reactions.

Hereinafter, a microfluidic structure suitable for a blood test formed in the substrate 1 will be described in detail. A first chamber 10 is formed in the substrate 1. The first chamber 10 receives a liquid sample, such as blood or serum. A second chamber 20 contains a first reagent in a liquid state that is used to dilute the sample to have a desired concentration suitable for examinations. The first reagent may be, for example, a buffer or distilled water (DI). A third chamber 30 contains a second reagent which is suitable for a reaction for detecting a target material contained in the sample. The first chamber 10 is connected to the second chamber 20 by a channel 41. The second chamber 20 is connected to the third chamber 30 by a channel 42. The chambers are fluid communicate with each other through channels 41 and 42 which allow a liquid flow between the chambers. The channels 41 and 42 contain valves 51 and 52, respectively. The valves 51 and 52 are used to control flow of a fluid flowing through the channels 41 and 42. Although not illustrated, the substrate 1 may be provided with inlets for loading the sample, the first reagent, and the second reagent; and an air vent for discharging air. In the microfluidic device 100 according to the current exemplary embodiment, the third chamber 30 is also adapted to, in addition to be adapted to store the second reagent, detect the target material contained in the sample, and thus, at least a portion of the substrate 1 corresponding to the third chamber 30 is transparent so that light can be transmitted therethrough, when the detection is made by optical measurements.

Various types of microfluidic valves may be used as the valves 51 and 52. For example, the valves 51 and 52 can be capillary valves that are manually opened when a pressure applied is increased to a predetermined level, or valves that are actively operated when an operation signal is transmitted and an operating power is externally provided. In the current exemplary embodiment, the valves 51 and 52 are formed of a valve forming material that absorbs electromagnetic radiation irradiated from an energy source to operate as a valve. The valves 51 and 52 are, so called "normally closed" valves that close the channels 41 and 42 to prevent flow of a fluid when energy of the electromagnetic radiation is not applied thereto.

The valve forming material may be a thermoplastic resin, such as a cyclic olefin copolymer (COC), polymethylmethacrylate (PMMA), polycarbonate (PC), polystyrene (PS), polyoxymethylene (POM), perfluoralkoxy (PFA), polyvinylchloride (PVC), polypropylene (PP), polyethylene terephthalate (PET), polyetheretherketone (PEEK), polyamide (PA), polysulfone (PSU), or polyvinylidene fluoride (PVDF).

The valve forming material can also be a phase transition material that exists in a solid state at room temperature. The phase transition material is loaded in its liquid state into the channels 41 and 42, and is then solidified to close the channels 41 and 42. The phase transition material may be wax. When heated, wax melts into a liquid and the volume thereof increases. The wax may be, for example, paraffin wax, microcrystalline wax, synthetic wax, or natural wax. The phase transition material may be gel or a thermoplastic resin. The gel may be selected from polyacrylamides, polyacrylates, polymethacrylates, and polyvinylamides.

The phase transition material contains a plurality of heat dissipating microparticles that absorb energy of electromagnetic radiation and dissipate thermal energy, which are dispersed in the phase transition material. The diameter of the heat dissipating particles may be in a range of 1 nm to 100 µm so that the heat dissipating particles freely pass through the channel 41 and 42 having a depth of about 01 mm and a width of 1 mm. When electromagnetic energy of, for example, a laser ray, is supplied, the temperature of the heat dissipating particles increases accordingly, and thus, the heat dissipating particles dissipate heat and become uniformly dispersed in the wax. Individual heat dissipating particle has a core which may contain a metal, and a hydrophobic shell. For example, the heat dissipating particle may have a core formed of Fe, and a plurality of surfactants that are bonded to and cover the Fe core. The heat dissipating particles may be stored as a dispersion in a carrier oil. The carrier oil may be hydrophobic so that the heat dissipating particles having a hydrophobic surface structure are uniformly dispersed. The carrier oil in which the heat dissipating particles are dispersed is mixed with a molten phase transition material, and the obtained mixture is loaded into the channels 41 and 42 and solidified, thereby closing the channels 41 and 42.

The heat dissipating particles may be, in addition to polymer particles described above, quantum dots or magnetic beads. The heat dissipating particles can also be micro particles of metal oxide, such as Al₂O₃, TiO₂, Ta₂O₃, Fe₂O₃, Fe₃O₄ or, HfO₂. However, it is not necessary to include the heat dissipating particles in the valves 51 and 52. For example, the valves 51 and 52 may be formed of only a phase transition material. The substrate 1 may be transparent at a location where the valves 51 and 52 are formed so that an electromagnetic radiation may be applied to the valves 51 and 52.

The second reagent for a blood test contained in the third chamber 30 may be a reagent suitable for reactions that facilitate detecting, for example, serum, aspartate aminotransferase (AST), albumin (ALB), alkaline phosphatase (ALP), alanine aminotransferase (ALT), amylase (AMY), urea nitrogen (BUN), calcium (Ca++), total cholesterol (CHOL), creatin kinase (CK), chloide (Cl-), creatinine (CREA), direct bilirubin (D-BIL), gamma glutamyl transferase (GGT), glucose (GLU), high-density lipoprotein cholesterol (HDL), potassium (K⁺), lactate dehydrogenase (LDH), low-density lipoprotein cholesterol (LDL), magnesium (Mg), phosphorus (PHOS), sodium (Na⁺), total carbon dioxide (TCO₂), total bilirubin (T-BIL), triglycerides (TRIG), uric acid (UA), albumin (ALB), or total protein (TP). In addition, it would be obvious to one of ordinary skill in the art that the microfluidic device can also be used to test, in addition to blood, various samples collected from a human body or any organisms.

The second reagent is lyophilized. The lyophilized reagent may in a form of beads. The amount of the second reagent in sold beads form to be loaded may not be adjusted as accurate as loading a liquid reagent. For example, it is difficult to produce the lyophilized second reagent beads of a uniform size and the produced second reagents beads are usually fragile. In addition, during the loading and storage of the second reagent beads in the third chamber 30, when the beads are exposed to humidity, the second reagent may lose at least partially its activity. In an exemplary embodiment, the second reagent contained in the third chamber 30 of the microfluidic device 100 is a solid reagent that is lyophilized in a solid state in an accurate amount, and optimized according to the purpose of an analysis to be performed. That is, at least one portion of the shape of the lyophilized second reagent is identical to or fits a portion of the internal configuration of the third chamber 30. The second reagent having the shape described above may be formed using the following method.

First, the second reagent in a liquid state is loaded into the third chamber 30 of the microfluidic device 100. The second reagent may be loaded into the third chamber 30 through an inlet (not shown). Alternatively, the second reagent in a liquid state can be directly loaded into the third chamber 30 defined by the bottom plate 11, or the bottom plate 11 and the intermediate plate 13, before the bottom plate 11 is bonded to the top plate 12.

By increasing the concentration of the second reagent in a liquid state, the volume of the second reagent loaded into the third chamber may be reduced. The amount of the liquid second reagent loaded into the third chamber 30 may be accurately adjusted, and a volume coefficient of variation of the second reagent may be within 3%.

A filler may be added to the liquid second reagent. When a filler is contained in the second reagent, the second reagent has a porous structure when lyophilized. Therefore, when a mixture of the liquid sample and the first reagent is introduced into the third chamber 30, the lyophilized second reagent may be easily dissolved. The filler may be at least one material selected from the group consisting of bovine serum albumin (BSA), polyethylene glycol (PEG), dextran, mannitol, polyalcohol, myo-inositol, an citric acid, ethylene diamine tetra acetic acid disodium salt (EDTA2Na), and polyoxyethylene glycol dodecyl ether (BRIJ-35). The filler may be properly chosen according to the type of the second reagent.

A surfactant may be added to the liquid second reagent. For example, the surfactant may be at least one material selected from the group consisting of polyoxyethylene, lauryl ether, octoxynol, polyethylene alkyl alcohol, nonylphenol polyethylene glycol ether; ethylene oxid, ethoxylated tridecyl alcohol, polyoxyethylene nonylphenyl ether phosphate sodium salt, and sodium dodecyl sulfate. The surfactant may be properly selected according to the type of the second reagent.

A microfluidic device 100 which contains the second reagent described above may be fabricated by, for example, subjecting the bottom plate 11 of FIG. 2 or the bottom plate 11 coupled to the intermediate plate 13, which each contains an accurate amount of the liquid second reagent in the third chamber 30to lyophilization under appropriate conditions, prior to coupling it to the top plate 12. The lyophilizing method includes a freezing process whereby water included in a material is frozen and a drying process whereby the frozen water is removed. In general, the drying process uses a sublimating process whereby frozen water is directly changed into a vapor. However, the entire drying process does not necessarily require sublimation, that is, only a part of the drying process may require sublimation. To perform the sublimating process, the pressure in the drying process may be adjusted to be equal to or lower than the triple point of water (6 mbar or 4.6 Torr). However, there is no need to maintain a constant pressure. In the drying process, the temperature may be changed. For example, after the freezing process is completed, the temperature may be gradually increased.

Through the processes described above, the microfluidic device 100 having the structure illustrated in FIGS. 2 and 3 can be manufactured. That is, at least a portion of the shape of the lyophilized solid second reagent is identical to or fits at least a portion of the internal configuration of the third chamber 30. The microfluidic device 100 according to the current exemplary embodiment can be used to accurately control the loading amount of the second reagent because the second reagent is loaded in a liquid state to the third chamber 30 of the microfluidic device 100. In addition, since the lyophilizing method is used after the liquid second reagent is loaded into the microfluidic device 100, the mass production of microfluidic devices for analyzing the same target material is possible.

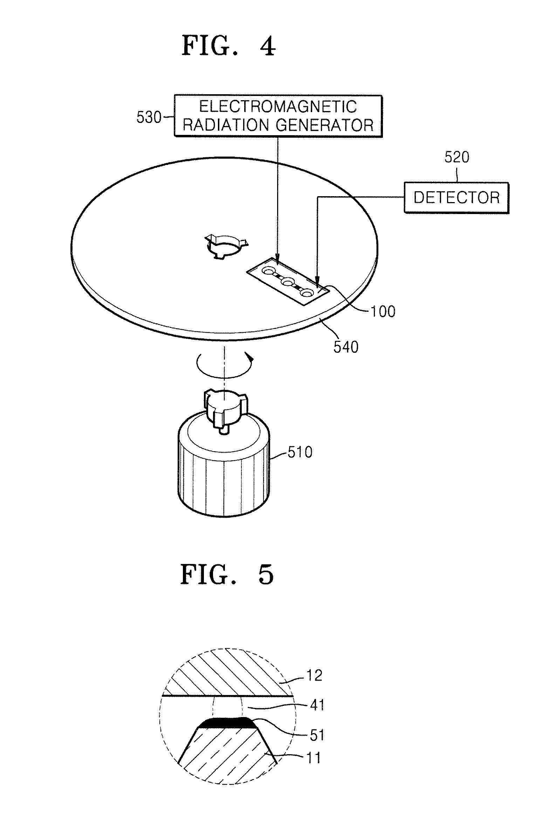

FIG. 4 is a schematic view of an analyzer including the microfluidic device 100 of FIG. 1. Referring to FIGS. 1 and 4, a rotary driving unit 510 rotates the microfluidic device 100 and mixes the sample, the first reagent, and the second reagent by a centrifugal force. The rotary driving unit 510 moves the microfluidic device 100 to a predetermined position so that the third chamber 30 faces a detector 520. The rotary driving unit 510 may further include a motor drive device (not shown) for controlling an angular position of the microfluidic device 100. The motor drive device may use a step motor or a direct-current motor. The detector 520 detects, for example, optical characteristics, such as fluorescent, luminescent, and/or absorbent characteristics, of a material to be detected. An electromagnetic radiation generator 530 is used to operate the valves 51 and 52, and emits, for example, a laser beam.

A method of analyzing the sample will now be described in detail. The first reagent, such as a buffer or distilled water, is loaded into the second chamber 20 of the microfluidic device 100 in which the second reagent lyophilized to be in a solid state has been stored in the third chamber 30 in advance, and then, the sample, such as serum, taken from a subject to be examined is loaded into the first chamber 10.

Then, the microfluidic device 100 is installed in the analyzer illustrated in FIG. 4. When the microfluidic device 100 is chip-shaped and cannot be directly mounted on the rotary driving unit 510, the microfluidic device 100 is inserted to an adaptor 540 and the adaptor 540 is mounted on the rotary driving unit 510. In this regard, since a fluid flows from the first chamber 10 to the third chamber 30, the microfluidic device 100 may be inserted in a way that the first chamber 10 is positioned closer to a rotary center of the adaptor 540 than the third chamber 30 is. The rotary driving unit 510 rotates the microfluidic device 100 so that the valve 51 faces the electromagnetic radiation generator 530. When electromagnetic radiation is irradiated on the valve 51, a material that forms the valve 51 melts by energy of electromagnetic radiation and the channel 41 is opened as illustrated in FIG. 5. The sample passes through the channel 41 by a centrifugal force and flows to the second chamber 20. The rotary driving unit 510 laterally shakes the microfluidic device 100 to mix the sample with the first reagent to form a sample mixture. Then, electromagnetic radiation is irradiated on the valve 52 to open the channel 52 and the sample mixture is loaded into the third chamber 30. Then, the rotary driving unit 510 laterally shakes the microfluidic device 100 a few times to dissolve the lyophilized second reagent by mixing it with the sample mixture. Therefore, a reagent mixture is formed in the third chamber 30.

Then, the third chamber 30 is moved to face the detector 520 so as to identify whether a material to be detected is present in the reagent mixture in the third chamber 30, and to measure the amount of the detected material, thereby completing the sample analysis.

As described above, an operator may perform a sample analysis by loading a sample to the microfluidic device 100 in which the lyophilized second reagent is loaded in advance and then mounting the resultant microfluidic device 100 on the analyzer.

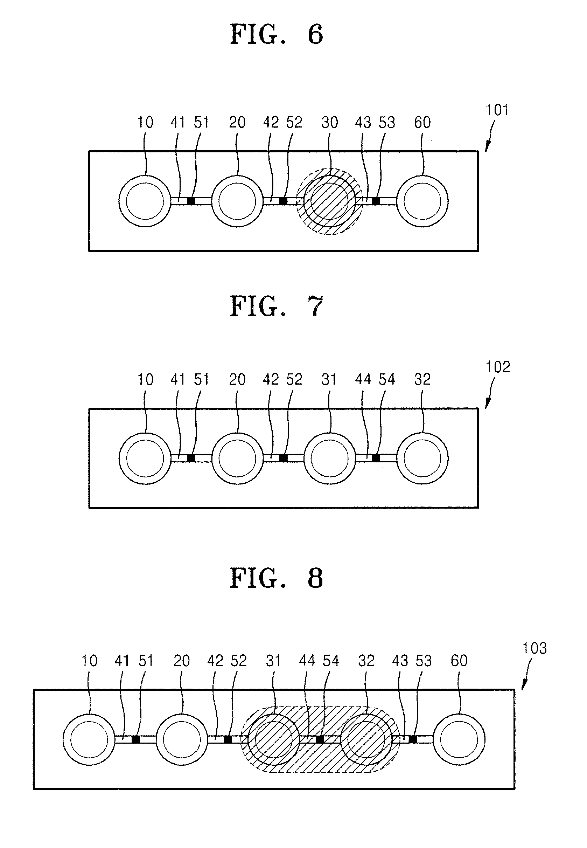

FIG. 6 is a plan view of a microfluidic device 101 according to another embodiment of the present invention. Referring to FIG. 6, the microfluidic device 101 has the same structure as the microfluidic device 100 illustrated in FIG. 1, except that the third chamber 30 is non-transparent and a detection chamber 60 connected to the third chamber 30 is further included. If a second reagent to be employed is susceptible to light, the second reagent will need to be protected from exposure to light. Therefore in this case, the third chamber 30 is formed to be non-transparent so that light does not pass therethrough. For example, as illustrated in FIG 6, a material which does not transmit light may be coated on an area including the third chamber 30 defined by a dotted line. The detection chamber 60 is transparent so that light can pass therethrough. The third chamber 30 is connected to the detection chamber 60 by a channel 43, and the channel 43 includes a valve 53. The valve 53 may be a valve that operates based on the same principle as that of the valves 51 and 52.

Due to the structure described above, the lyophilized second reagent is not exposed to light when not used, and a sample analysis process is performed using a mixed fluid including the sample, the first reagent, and the second reagent in the detection chamber 60.

FIG. 7 is a plan view of a microfluidic device 102 according to another embodiment of the present invention. Referring to FIG. 7, the microfluidic device 102 illustrated in FIG. 7 has the same structure as the microfluidic device 100 illustrated in FIG. 1, except that the third chamber 30 is replaced with two sub-chambers 31 and 32.

In some cases, a second reagent may contain a component that degrades the activity of the second reagent when the component is mixed and lypophilized. In such case, for example, when the reagent is composed of an enzyme and a substrate of the enzyme, second reagent and the elements need to be separated from each other. Examples of such a reagent include a reagent for detecting alanine phosphatase (ALP), a reagent for detecting alanine aminotransferase (ALT), a reagent for detecting high-density lipoprotein cholesterol (HDL), and a reagent for detecting low-density lipoprotein cholesterol (LDL). When a material acting as a substrate and enzyme co-exist in a biochemical reaction, titer may be degraded. Therefore, the substrate should be separated from enzyme. Specifically, for ALP, p-nirophenolphosphate (PNPP), a substrate, is unstable at pH 10 or higher, and aminomethanpropanol (AMP) and diethanolamin (DEA) each acting as buffer that is necessary in a reaction system has a pH of 11-11.5. Therefore, the substrate and the buffer should be independently lyophilized.

In addition, a reagent for detecting an amylase (AMY) includes a buffer and a substrate. However, when the reagent is used to detect AMY, NaCl is necessary. However, NaCl has deliquescent characteristics and thus it is difficult to lyophilize NaCl. Even when NaCl is lyophilized, the lyophilized NaCl immediately absorbs humidity and the shape thereof is changed, and titer may be degraded. Therefore, NaCl should be separated from the buffer and the substrate. Therefore, in such case, a first component of the second reagent and a second component of the second reagent are respectively loaded into the sub-chambers 31 and 32 in liquid states, and then lyophilized.

The sub-chambers 31 and 32 are connected by a channel 44, and the channel 44 includes a valve 54. The valve 54 may be a value that operates based on the same principle as that of the valves 51 and 52. Also, since a sample, a first reagent, and the first component, and the second component are mixed in the sub-chamber 32, the sub-chamber 32 acts as a detection chamber. According to the exemplary current embodiment, the number of sub-chambers 31 and 32 is two, but is not limited thereto. For example, depending on the type and characteristics of a second reagent used, the number of sub-chambers may be three or more.

FIG. 8 is a plan view of a microfluidic device 103 according to another embodiment of the present invention. Referring to FIG. 8, the microfluidic device 103 has the same structure of the microfluidic device 101 illustrated in FIG. 6, except that the non-transparent third chamber 30 is replaced with two non-transparent sub-chambers 31 and 32. As described above, when the second reagent includes a first component and a second component which may degrade activity of the second reagent when lyophilized together and are susceptible to light, the first component and the second component may be respectively loaded into non-transparent sub-chambers 31 and 32 and then lyophilized. An analyzing process may be performed using a mixed fluid including a sample, a first reagent, the first component of the second reagent, and the second reagent component of the second reagent in a detection chamber 60. In the current embodiment, the number of sub-chambers 31 and 32 is two, but is not limited thereto. For example, according to the type and characteristics of a second reagent used, the number of sub-chambers may be three or more.

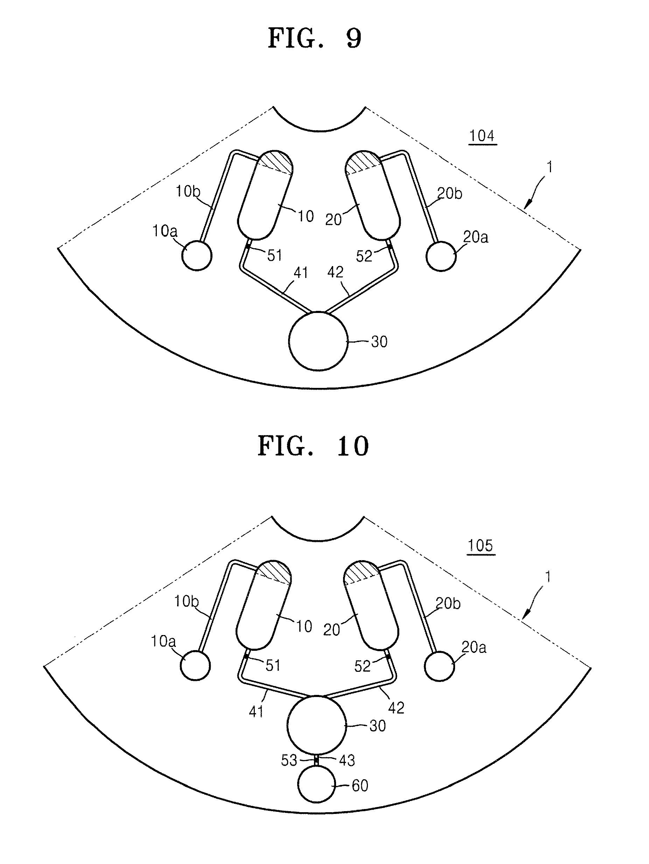

FIG. 9 is a plan view of a microfluidic device 104 according to another embodiment. Referring to FIGS. 2, 3, and 9, the microfluidic device 104 according to the current embodiment is disc-shaped and can be directly mounted on the rotary driving unit 510 of the analyzer (see FIG. 4). Although only a part of the microfluidic device 104 is illustrated in FIG. 9, the substrate 1 is disc-shaped. The substrate 1 may have the two-layer structure illustrated in FIG 2 or the three-layer structure illustrated in FIG 3.

The substrate 1 includes a first chamber 10, a second chamber 20, and a third chamber 30. The third chamber 30 may be farther from a rotary center of the substrate 1 than the first chamber 10 and the second chamber 20. A channel 41 extends from the first chamber 10 and is connected to third chamber 30. The channel 42 extends from the second chamber 20 and is connected to the third chamber 30. The channels 41 and 42 include valves 51 and 52, respectively.

A sample discharge chamber 10a accommodates excess sample loaded into the first chamber 10. The first chamber 10 and the sample discharge chamber 10a are connected by a channel 10b. The channel 10b may include a capillary valve. A first reagent discharge chamber 20a accommodates excess first reagent loaded into the second chamber 20. The second chamber 20 and the first reagent discharge chamber 20a are connected by a channel 20b. The channel 20b may include a capillary valve.

The third chamber 30 contains a second reagent that is lyophilized. As described above, the bottom plate 11 of FIG. 2 or the bottom plate 11 coupled to the intermediate plate 13, which each contain an accurate amount of the liquid second reagent in the third chamber 30, are loaded into a lyophilizing device and then an appropriate method is employed to freeze dry the second reagent in the third chamber 30. In some cases, bonding with the top plate 11 may be further performed. Therefore, at least a portion of the shape of the lyophilized second reagent is identical to or fits at least a portion of the internal configuration of the third chamber 30.

A method of analyzing a sample will now be described in detail with reference to FIGS. 4 and 9. The first reagent, such as a buffer or distilled water, is loaded into the second chamber 20 of the microfluidic device 104 in which the lyophilized solid second reagent is stored in advance. In this case, a sufficiently large amount of the first reagent is loaded into the second microfluidic device 20. Then, the sample, such as blood taken from a subject to be examined or serum isolated from the blood, is loaded into the first chamber 10. In this case, sufficiently large amount of the sample is loaded into the first chamber 10.

Then, the microfluidic device 104 is mounted on the rotary driving unit 510 of the analyzer (see FIG. 4). The rotary driving unit 510 rotates the microfluidic device 104 and a portion of the sample contained in the first chamber 10 is discharged to the sample discharge chamber 10a through a channel 10b. The sample discharge chamber 10a may be located to radially farther from a rotary center of the substrate 1 than the first chamber 10, and fluid communicates with the first chamber 10 through the channel 10b. The channel 10b may be connected to the first chamber 10 at an appropriate position of the first chamber 10 in a way to adjust the amount maintained in the first chamber 10. That is, a portion of the sample contained in a hatched portion of the first chamber 10 (i.e., portion which has a same or shorter distance from the rotary center than the channel 10b) passes through the capillary valve and the channel 10b and is then discharged to the sample discharge chamber 10a. A second chamber 20, a discharge channel 20b and a discharge chamber 20a may have the substantially same configuration to one described above. The amounts of the sample and the first reagent can be accurately adjusted and then sample analysis can be performed.

Then, the rotary driving unit 510 rotates the microfluidic device 104 so that the valves 51 and 52 face the electromagnetic radiation generator 530. When electromagnetic radiation is irradiated on the valves 51 and 52, a material forming the valves 51 and 52 melts and the channels 41 and 42 are opened. When the microfluidic device 104 is rotated, the sample and the first reagent are loaded into the third chamber 30 through the channels 41 and 42 by a centrifugal force. The lyophilized second reagent is mixed with a sample mixture including the sample and the first reagent and dissolved. The rotary driving unit 510 may shake the microfluidic device 104 a few times to dissolve the lyophilized second reagent, thereby preparing a reagent mixture.

Then, the third chamber 30 is moved to face the detector 520 so as to identify whether a material to be detected is present in the reagent mixture in the third chamber 30, and to measure the amount of the detected material, thereby completing the sample analysis.

FIG. 10 is a plan view of a microfluidic device 105 according to another embodiment of the present invention. Referring to FIG. 10, the microfluidic device 105 has the same structure as the microfluidic device 104 illustrated in FIG. 9, except that the third chamber 30 is non-transparent, a detection chamber 60 connected to the third chamber 30 is further formed. The third chamber 30 is non-transparent so that light cannot pass the third chamber 30 so that a second reagent that is susceptible to light is not exposed to light when in the third chamber 30. For example, as described above, a portion of the substrate 1 corresponding to the third chamber 30 can be coated with a non-transparent material. The detection chamber 60 is transparent so that light can pass through the detection chamber 60. The third chamber 30 may be connected to the detection chamber 60 by the channel 43, and the channel 43 includes a valve 53. The lyophilized second reagent is not exposed to light when not used, and a sample analysis process is performed using a mixed fluid including the sample, the first reagent, and the second reagent in the detection chamber 60.

Although not illustrated in FIGS. 9 and 10, the third chamber 30 can be replaced with the sub-chambers 31 and 32 illustrated in FIGS. 7 and 8, which may be obvious to one of ordinary skilled in the art.

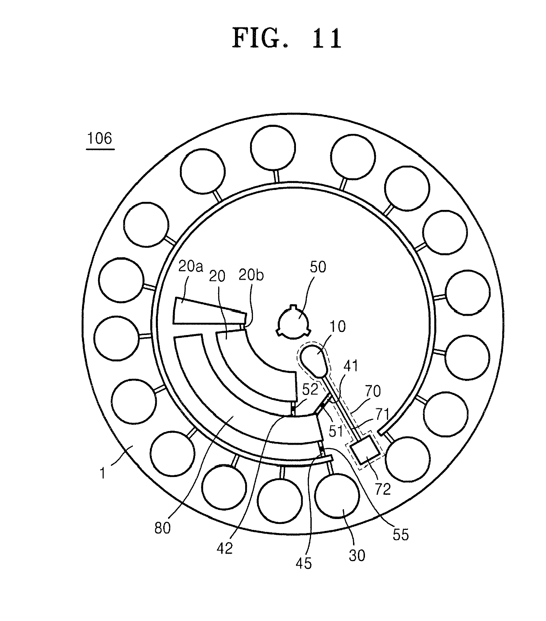

FIG. 11 is a plan view of a microfluidic device 106 according to another exemplary embodiment. Referring to FIG 11, the microfluidic device 106 according to the current embodiment is disc-shaped and can be directly mounted on the rotary driving unit 510 of the analyzer (see FIG. 4). The microfluidic device 106 is provided with a centrifuging unit 70 for isolating a supernatant from a sample. For example, when whole blood is loaded as a sample, the centrifuging unit 70 separates the whole blood into serum and precipitations. The substrate 1 is disk-shaped. The substrate 1 may have the two-layer structure illustrated in FIG. 2 or the three-layer structure illustrated in FIG 3.

Hereinafter, a portion of the substrate 1 being close to a center of the substrate 1 will be referred to as an inner portion, and a portion of the substrate 1 being far from the center will be referred to as an outer portion. The first chamber 10 is closer to the center of the substrate 1 than any other elements that form the microfluidic device 106. The centrifuging unit 70 includes a centrifuging portion 71 positioned outside the first chamber 10 and a precipitations collector 72 positioned at an end of the centrifuging portion 71. When a sample is centrifuged, the supernatant remains in the first chamber 10 or flows to the centrifuging portion 71, and precipitations or a liquid portion having a relatively greater gravity flow to the precipitations collector 72. A channel 41 is positioned on the side of the centrifuging portion 71 and guides the isolated supernatant to a mixing chamber 80. The channel 41 includes a valve 51.

A second chamber 20 contains a first reagent. The second chamber 20 is connected to the mixing chamber 80 by a channel 42. A first reagent discharge chamber 20a discharges excess first reagent loaded into the second chamber 20. The second chamber 20 and the first reagent discharge chamber 20a are connected by a channel 20b. The channel 20b may include a capillary valve.

A plurality of third chambers 30 are positioned along a circumferential direction of the substrate 1. The mixing chamber 80 is connected to the third chambers 30 by a channel 45. The channel 45 includes a valve 55. The valve 55 may be formed of a valve forming material that changes state when exposed to electromagnetic radiation. Each of the third chambers 30 may contain the same or different second reagent that is lyophilized. For example, a liquid second reagent may be loaded into each third chamber 30, and the substrate 1 is loaded into a lyophilizer to perform a lyophilizing process. Therefore, at least a portion of the shape of the lyophilized second reagent contained in the microfluidic device 105 is identical to or fits at least a portion of the internal configuration of the third chamber 30.

A method of analyzing a sample will now be described in detail with reference to FIGS. 4 and 11. The first reagent, such as a buffer or distilled water, is loaded into the second chamber 20 of the microfluidic device 106 in which the lyophilized solid second reagent is stored in advance. In this case, a sufficiently large amount of the first reagent is loaded into the second chamber 20. Then, a sample, such as blood taken from a subject to be examined or serum isolated from the blood, is loaded into the first chamber 10.

Then, the microfluidic device 106 is mounted on the rotary driving unit 510 of the analyzer (see FIG. 4). The rotary driving unit 110 rotates the microfluidic device 104 and thus, a supernatant of the sample contained in the first chamber 10 remains in the first chamber 10 or flows to the centrifuging portion 71 by a centrifugal force and high gravity precipitations flow to the precipitations collector 72. In addition, a portion of the first reagent contained in a portion of the second chamber 20 closer to a rotary center of the substrate 1 than a portion of the second chamber 20 connected to the channel 20b passes through the capillary valve and the channel 20b and is discharged to the first reagent discharge chamber 20a. Due to the operation described above, the amounts of the sample and the first reagent can be accurately adjusted and then a sample analysis can be performed.

Then, the rotary driving unit 510 moves the microfluidic device 106 so that the valves 51 and 52 face the electromagnetic radiation generator 530. When electromagnetic radiation is irradiated on the valves 51 and 52, a valve forming material that forms the valves 51 and 52 melts due to energy of electromagnetic radiation, and the channels 41 and 42 are opened. When the microfluidic device 106 is rotated, the sample and the first reagent are flow into the mixing chamber 80 by a centrifugal force through the channels 41 and 42, thereby forming a sample mixture including the sample and the first reagent in the mixing chamber 80. To mix the sample with the first reagent, the rotary driving unit 510 may laterally shake the microfluidic device 106 a few times.

Then, the rotary driving unit 510 moves the microfluidic device 106 so that the valve 55 faces the electromagnetic radiation generator 530. When electromagnetic radiation is irradiated on the valve 55, a valve forming material that forms the valve 55 melts due to energy of electromagnetic radiation and the channel 45 is opened. When the microfluidic device 106 rotates, the sample mixture is loaded into the third chamber 30 through the channel 45. The lyophilized second reagent is mixed with the sample and the first reagent and dissolved, thereby forming a reagent mixture. To dissolve the lyophilized second reagent, the rotary driving unit 510 may laterally shake the microfluidic device 106 a few times.

Then, the third chamber 30 is moved to face the detector 520 so as to identify whether a material to be detected is present in the reagent mixture in the third chamber 30, and to measure the amount of the detected material, thereby completing the sample analysis.

Although not illustrated in FIG. 11, the third chamber 30 of the microfluidic device 106 can be replaced with the sub-chambers 31 and 32 illustrated in FIGS. 7 and 8 and the detection chamber 60 illustrated in FIG. 10 can be further included, which is obvious to one of ordinary skill in the art.

As described above, the microfluidic device and the method of analyzing a sample using the microfluidic device allow an operator can perform a blood test by merely loading a sample and a first reagent to the microfluidic device in which a lyophilized second reagent is contained in advance and mounting the reagent on an analyzer.

In addition, the blood test can be performed without rotating a microfluidic device. For example, in the chip-shaped microfluidic devices 100-103 illustrated in FIGS. 1-8, the valves 51, 52, 53 and 54 can be opened using electromagnetic radiation, the operator can directly shake the microfluidic devices to prepare a reagent mixture, the chip-shaped microfluidic devices 100-103 can then be mounted on an analyzer, and then a detector may be used.

As described above, the microfluidic devices described above can be manufactured without a great amount of effort to simultaneously form lyophilized second reagent beads of uniform sizes, and to large quantities, and without any difficulty for loading the lyophilized second reagent beads to the microfluidic device. In addition, a microfluidic device in which the second reagent is contained in a lyophilized state in advance can easily be mass-produced and thus, the manufacturing costs are low and high compatibility can be obtained.

While the present invention has been particularly shown and described with reference to exemplary embodiments thereof, it will be understood by those of ordinary skill in the art that various changes in form and details may be made therein without departing from the scope of the present invention as defined by the following claims.

## Claims

1. A microfluidic device (100-106) comprising:
a substrate (1) comprising a plurality of chambers (10, 10a, 20, 20a, 30, 31, 32, 60);
a solid reagent, which is a lyophilized reagent, contained in at least one of the plurality of chambers, wherein at least a portion of the shape of the solid reagent is identical to at least a portion of the configuration of the inner surface of the at least one chamber.

2. The microfluidic device of claim 1, further comprising:
a plurality of channels (41, 42, 43, 44, 52, 10b, 20b) connecting the plurality of chambers; and
a valve (51, 52, 53, 54, 55), included in at least one of the plurality of channels, which controls flow of a fluid through the plurality of channels, in particular, wherein the valve is formed of a valve forming material that changes its state when exposed to electromagnetic radiation, and the phase change results in opening of the valve.

3. The microfluidic device of claim 2, wherein the valve forming material is selected from a phase transition material and a thermoplastic resin, wherein the phase of the phase transition material or the thermoplastic resin changes when exposed to energy of the electromagnetic radiation.

4. The microfluidic device of claim 3, wherein the valve forming material comprises heat dissipating particles which are dispersed in the phase transition material, and absorb energy of the electromagnetic radiation and dissipate the energy.

5. The microfluidic device of any preceding claims, wherein solid reagent comprises at least one reagent selected from the group consisting of reagents for detecting serum, aspartate aminotransferase (AST), albumin (ALB), alkaline phosphatase (ALP), ala aminotransferase (ALT), amylase (AMY), urea nitrogen (BUN), calcium (Ca++), total cholesterol (CHOL), creatin kinase (CK), chloride (Cl-), creatinine (CREA), direct bilirubin (D-BIL), gamma glutamyl transferase (GGT), glucose (GLU), high-density lipoprotein cholesterol (HDL), potassium (K+), lactate dehydrogenase (LDH), low-density lipoprotein cholesterol (LDL), magnesium (Mg), phosphorus (PHOS), sodium (Na+), total carbon dioxide (TCO₂) total bilirubin (T-BIL), triglycerides (TRIG), uric acid (UA), albumin (ALB), and total protein (TP).

6. The microfluidic device of any preceding claims, wherein the solid reagent comprises at least one of a filler and a surfactant.

7. The microfluidic device of claim 6, wherein the filler comprises at least one material selected from the group consisting of bovine serum albumin (BSA), polyethylene glycol (PEG), dextran, mannitol, polyalcohol, myo-inositol, an citric acid, ethylene diamine tetra acetic acid disodium salt (EDTA2Na), and polyoxyethylene glycol dodecyl ether, and
wherein the surfactant comprises at least one material selected from the group consisting of polyoxyethylene, lauryl ether, octoxynol, polyethylene alkyl alcohol, nonylphenol polyethylene glycol ether; ethylene oxid, ethoxylated tridecyl alcohol, polyoxyethylene nonylphenyl ether phosphate sodium salt, and sodium dodecyl sulfate.

8. The microfluidic device of any preceding claims, wherein the solid reagent is prepared by condensing a reagent to have a concentration higher than a concentration that is suitable for an analysis of the sample and lyophilizing the condensed reagent.

9. The microfluidic device of any preceding claims, wherein the plurality of chambers comprises a first chamber to contain a liquid sample to be analyzed, a second chamber to contain a liquid reagent, and a third chamber which contains the solid reagent.

10. The microfluidic device of claim 9, wherein the third chamber is a detection chamber that is used to detect a target material contained in the sample, in particular, wherein the detection chamber is a transparent chamber.

11. The microfluidic device of claim 9, wherein the third chamber comprises a plurality of sub-chambers, wherein
a plurality of solid reagent components are respectively contained in a lyophilized state in the plurality of sub-chambers, wherein
when the plurality of solid reagent components are mixed and lyophilized, activity of the plurality of solid reagent components degrades.

12. The microfluidic device of claim 9, further comprising:
a transparent detection chamber connected to the third chamber, wherein
the third chamber is non-transparent so that light does not pass through the third chamber.

13. A method of analyzing a sample using a microfluidic device comprising plural chambers connected by a plurality of channels each comprising a valve, the method comprising:
providing a microfluidic device of which chamber (I) contains a solid lyophilized reagent (I);
loading a liquid reagent (II) into a chamber (II);
loading the sample into a chamber (III);
opening the valve and mixing the sample with the reagent (II) to form a sample mixture;
mixing the sample mixture with the lyophilized reagent (I) to form a reagent mixture; and
detecting a reaction of the reagent mixture in the chamber (I).

14. The method of claim 13, wherein at least a portion of the shape of the lyophilized reagent is identical to at least a portion of the shape of the chamber (I).

15. The method of claim 13 or 14, wherein the opening the valve comprises supplying electromagnetic energy to a valve forming material in the channel so that the valve forming material melts.
